# FASCICULE DE BREVET EUROPEEN

(11) **EP 2 545 958 B1**
(45) Date de publication et mention de la délivrance du brevet: **14.05.2014**
(21) Numéro de dépôt: 12164157.5
(22) Date de dépôt: 13.04.2012
(51) Int. Cl.: A61N 1/05, A61N 1/37, A61N 1/08

(54) **Sonde pour prothèse cardiaque implantable, comprenant des moyens de protection contre les effets thermiques des champs IRM**
Sonde für implantierbare Herzprothese, die Schutzmittel gegen die Wärmeeffekte von Magnetresonanz-Feldern umfasst
Probe for implantable cardiac prosthesis, comprising a means for protection against the thermal effects of MRI fields

(30) Priorité: 12.07.2011 FR 1156360
(43) Date de publication de la demande: 16.01.2013
(73) Titulaire: Sorin CRM SAS, 92140 Clamart (FR)
(72) Inventeur: Ollivier, Jean-François, 91190 Villiers Le Bacle (FR); D'Hiver, Philippe, 92320 Chatillon (FR)
(74) Mandataire: Dupuis-Latour, Dominique

(56) Documents cités:
- EP-A1- 2 436 422
- WO-A1-2011/137304
- US-A1- 2011 054 582
- US-A1- 2011 106 231

## Description

L'invention concerne les sondes intracardiaques de stimulation ou de défibrillation.

Ces sondes permettent le recueil de signaux de dépolarisation pour la surveillance en continu du rythme cardiaque et si nécessaire l'application d'impulsions électriques de stimulation, de resynchronisation et/ou de défibrillation. Elles sont couplées à un générateur implanté, avec lequel elles forment un "dispositif médical implantable actif" tel que défini par la Directive 90/385/CEE du 20 juin 1990 du Conseil des communautés européennes.

L'invention concerne avantageusement les sondes pourvues à leur extrémité distale d'une vis permettant l'ancrage de la tête de sonde dans le tissu de l'endocarde au point de contact avec ce dernier. En outre, dans le cas d'une vis dite "active", une fois en place la vis joue le rôle d'électrode distale de détection/stimulation du myocarde.

Une telle sonde, dotée d'une vis rétractable, est notamment divulguée par le EP 0 591 053 A, qui décrit un type de sonde actuellement commercialisée sous la dénomination *Stelix* (marque déposée) par Sorin CRM, Clamart, France.

Ces sondes peuvent être des sondes endocavitaires (placées dans une cavité du myocarde en contact avec la paroi de celui-ci), épicardiques (notamment pour définir un potentiel de référence, ou pour appliquer un choc), ou encore intravasculaires (la sonde est par exemple introduite dans le sinus coronaire jusqu'à un emplacement situé face à la paroi du ventricule gauche).

L'invention est toutefois applicable à d'autres types de sondes, par exemple celles dont l'électrode distale reste en surface de la paroi musculaire, et qui sont alors pourvues de barbes d'ancrage pour assurer leur maintien en place au site choisi. Les EP 0 784 993 A1 et EP 0 779 08 A1 (ELA Medical) décrivent des exemples de telles sondes à barbes. L'invention concerne plus particulièrement les techniques permettant de sécuriser les sondes lorsque le porteur doit être soumis à un examen par imagerie par résonance magnétique (IRM ou MRI).

En effet, un examen IRM est aujourd'hui contre-indiqué aux patients porteurs d'un stimulateur ou défibrillateur cardiaque. Plusieurs types de problèmes en sont la cause :
- un échauffement à proximité des électrodes de la sonde reliées au générateur ;
- les forces et couples d'attraction exercés sur le dispositif plongé dans le champ magnétique statique très élevé de l'appareil IRM ;
- un comportement imprédictible du dispositif lui-même, du fait de l'exposition à ces champs magnétiques extrêmes.

La présente invention a pour objet d'apporter une solution au premier type de problème.

Le problème d'échauffement apparaît surtout au voisinage des électrodes, situées à l'extrémité distale des sondes. En effet les sondes, placées dans l'imageur IRM, se comportent comme des antennes et captent le champ radiofréquence (RF) émis par l'imageur. Les sondes plongées dans ce champ RF voient dès lors circuler dans leurs conducteurs des courants induits provoquant autour des électrodes en contact avec le sang un échauffement des tissus environnants. L'échauffement au niveau des électrodes étant proportionnel à la densité de courant circulant dans celles-ci, plus la surface de l'électrode est faible (cas typique d'une vis active), plus la densité de courant est grande et donc plus l'échauffement des tissus environnants sera élevé.

En pratique, selon la configuration relative du générateur, des sondes et de l'imageur IRM, les élévations de température constatées expérimentalement atteignent typiquement 8°C (pour des électrodes en carbone) à 12°C (pour des électrodes en métal), parfois même jusqu'à 30 °C.

Or l'élévation de température ne doit pas être supérieure à ce qui est spécifié dans la norme EN 45502-1 et ses dérivés, soit moins que 2°C. En effet, à partir de 4°C survient une mort cellulaire locale qui a pour effet immédiat, entre autres, de modifier substantiellement et de manière irréversible les seuils de détection et de stimulation, voire d'entraîner une perte totale de la capture.

Il est certes possible, comme décrit notamment par les US 2003/0204217 A1 et US 2007/0255332 A1, de prévoir un mode de mise en sécurité IRM dans lequel un circuit de protection met à la masse tous les conducteurs pour empêcher la circulation de courants parasites induits.

Mais cette manière de procéder empêche le dispositif de rester fonctionnel pendant la durée de l'examen. Or cet examen peut se prolonger plusieurs minutes, et il est pour cette raison éminemment souhaitable que le dispositif puisse continuer à assurer sans discontinuité les fonctions de détection des potentiels de dépolarisation et de stimulation éventuelle du myocarde.

Pour réduire les courants induits sans pour autant déconnecter ou mettre à la masse des conducteurs, diverses techniques ont été proposées, reposant principalement sur la mise en série avec le ou les conducteurs d'une impédance s'opposant au passage du courant dans une situation d'examen IRM. Il peut s'agir d'un simple bobinage (cf. US 7 123 013 B2), ou d'un circuit résonant de type circuit bouchon accordé sur la fréquence RF de l'imageur (cf. les US 2011/0106231, US 2010/0208397 A1 et US 2011/0054582 A1). Il a également été proposé un circuit de protection passive consistant à placer une diode PIN en parallèle avec une résistance montée en série avec l'électrode (cf. US 2008/0154348 A1). Une autre approche encore, proposée par le EP 2 198 917 A1 (ELA Médical) consiste, dans le cas d'une sonde bipolaire, à déconnecter l'un des conducteurs et à le relier à la masse du boîtier du générateur, de manière que ce conducteur puisse faire fonction de blindage de protection de l'autre conducteur, resté fonctionnel.

Ces diverses solutions présentent cependant toutes un certain nombre de limitations, notamment :
- les systèmes mettant en oeuvre des commutations nécessitent pour leur activation un détecteur de champ magnétique dans le générateur ;
- les circuits de protection passive à circuit bouchon sont calculés pour une fréquence d'imageur spécifique ;
- les circuits de protection intégrés nécessitent parfois un conducteur supplémentaire dans la sonde et, par voie de conséquence, ne sont pas utilisables avec n'importe quel générateur sans modification matérielle des circuits de celui-ci ;
- les composants de protection, par exemple les diodes, sont susceptibles de provoquer des pertes substantielles d'énergie lors de l'envoi des impulsions de stimulation sur les électrodes ;
- l'ajout d'un circuit de protection intégré en partie distale du corps de sonde peut provoquer, lorsque le dispositif est baigné dans un champ IRM, la réflexion vers le générateur d'une tension RF qu'il sera nécessaire de filtrer et de dissiper ;
- l'incorporation d'un circuit de protection susceptible d'assurer en toutes circonstances l'autoprotection de la sonde est extrêmement délicate sur le plan technologique, compte tenu des contraintes physiques : diamètre extérieur limité à 5 French (1 F = 1/3 mm), nécessité d'une lumière interne dans le corps de sonde, limitation de la longueur de la partie rigide en tête de sonde, etc. ;
- le coût toujours élevé de la mise en oeuvre de ces technologies.

Un autre inconvénient tient au fait que le circuit de protection peut lui-même subir une élévation de température, transmise à l'électrode proximale et donc aux tissus de la paroi cardiaque.

Ainsi, dans le US 2011/0106231 A1 précité, le circuit bouchon résonant, qui bloque le passage du courant vers la vis d'ancrage formant électrode, comprend une inductance logée dans la tête de sonde. Lorsque l'ensemble est placé dans un champ IRM à une fréquence correspondant à celle du circuit bouchon, un courant élevé circule dans l'inductance et provoque ainsi un échauffement important à l'intérieur de la tête de sonde. Le document propose, pour évacuer cette chaleur vers l'extérieur, de munir la tête de sonde d'un chemisage extérieur dans la région de l'inductance, formant *heat spreader.* La chaleur produite au sein de l'inductance se diffuse radialement dans la tête de sonde puis au travers du chemisage, pour s'évacuer dans le flux sanguin environnant.

On soulignera que dans cette proposition le diffuseur thermique est placé au droit de l'inductance (c'est-à-dire à peu près au milieu de la partie terminale de la tête de sonde), et que l'extrémité distale de la tête de sonde n'est pas affectée par ce diffuseur. Plus précisément, cette extrémité distale est réalisée en un matériau souple de type silicone qui limiter la pression de contact sur les tissus. Le silicone est un mauvais conducteur de la chaleur, mais ceci n'est pas un inconvénient puisqu'en présence d'un champ RF la vis n'est plus alimentée (du fait du circuit bouchon) et les tissus ne s'échauffent donc pas à ce niveau.

On notera au surplus que la structure décrite dans ce document nécessite la présence d'une inductance relativement volumineuse, et qu'il est en outre indispensable de prévoir un isolement électrique entre cette inductance (qui est montée en série avec le conducteur de stimulation) et le diffuseur thermique (qui est nécessairement en contact avec le milieu sanguin environnant). Toutes ces contraintes augmentent la complexité de réalisation et le volume d'ensemble de la tête de sonde.

Les US 2010/0208397 A1 et US 2011/0054582 A1 précités décrivent des configurations de têtes de sonde comparables, avec un circuit bouchon de prévention contre les effets délétères d'un champ IRM, et un diffuseur thermique disposé au droit de l'inductance du circuit bouchon, de manière à permettre une évacuation, en direction radiale, de la chaleur générée au sein de l'inductance vers le milieu sanguin environnant.

Le but de l'invention est de remédier aux divers inconvénients rencontrés avec les circuits de protection de sonde connus, en proposant une nouvelle configuration de sonde à vis assurant une "autoprotection" permanente de la sonde à l'encontre des effets thermiques délétères des champs IRM - et ceci sans recours à aucun circuit de protection ou composant électronique additionnel.

Le but de l'invention est de proposer une telle configuration de sonde qui, en toutes circonstances, réduise notablement l'élévation de température à l'extrémité distale de la sonde lors d'un examen IRM et évite ainsi la destruction partielle ou totale des tissus avoisinant la vis d'ancrage.

Le point de départ de l'invention est la constatation du fait que la vis d'ancrage et son système de déploiement (dans le cas d'une vis rétractable) sont encapsulés dans un mécanisme dont les parois externes sont isolées électriquement, réalisées dans un matériau tel que polycarbonate, polyuréthanne, silicone ou PEEK (polyétheréthercétone).

Or le point commun à tous ces matériaux est leur faible conductivité thermique. L'enveloppe de la vis et de son système d'activation, généralement dénommée "boîtier" ou "*housing*" est donc non seulement un isolant électrique (ce qui est indispensable) mais également un isolant thermique. Il en résulte qu'en cas d'échauffement le flux thermique est confiné au centre de la vis, avec peu de possibilités de diffusion vers l'extérieur.

En effet, les échanges thermiques entre cette zone et les volumes adjacents sont limités :
- côté distal (pointe de la vis) et côté radial (périphérie de la partie émergée de la vis), par la faible conductivité du tissu musculaire dans lequel est implantée la vis, et
- côté proximal (côté du boîtier en contact avec la paroi cardiaque, mais à l'extérieur de celle-ci), par l'effet de "bouchon thermique" lié au matériau à faible conductivité du matériau du boîtier.

De ce fait, en cas d'échauffement de la vis sous l'effet d'un champ IRM intense l'élévation de température est d'autant plus accentuée que la chaleur générée n'a pas la possibilité de s'évacuer vers l'extérieur : ni dans la masse de la paroi du myocarde (peu conductrice thermiquement), ni dans le flux sanguin environnant (du fait de l'effet d'écran ou de bouchon thermique du boîtier enfermant la vis et son mécanisme).

Le WO 2011/137304 A1 propose, pour resoudre cette difficulté de doter l'extrémité de la sonde, au niveau du boîtier enfermant la vis d'ancrage et son mécanisme, d'un drain thermique le plus continu possible entre la surface extérieure du boîtier et le coeur de la vis fixée dans le tissu musculaire (là où se produit l'échauffement localisé), ceci afin d'utiliser au mieux l'effet de dissipation thermique générée par le flux sanguin autour du boîtier de la tête de sonde. Le flux thermique généré au niveau de la vis sera alors dissipé principalement en direction proximale, dans le flux sanguin via le boîtier thermiquement conducteur, limitant ainsi l'élévation de température au niveau des tissus.

Le EP 2 436 422 A1 décrit l'addition d'un hydrogel en bout de sonde pour accroitre encore l'évacuation de la chaleur produite.

La sonde de l'invention est du type général divulgué par le WO 2011/137304 A1 pricité, correspondant au préambule de la revendication 1. Pour améliorer le transport thermique au niveau de la vis d'ancrage, l'invention propose la caractéristique objet de la partie caractérisante de la revendication 1. Les sous-revendications visent des formes de réalisation subsidiaires, avantageuses.

On va maintenant décrire un exemple de mise en oeuvre du dispositif de l'invention, en référence au dessins annexés.
La Figure 1 représente la tête d'une sonde selon l'invention, en coupe longitudinale par un plan axial.
La Figure 2 est une vue agrandie de la partie droite de la Figure 1, montrant la manière dont s'opère le transfert de chaleur au sein de l'extrémité distale de la sonde en contact avec la paroi cardiaque.

On va maintenant décrire un exemple de réalisation de la sonde de l'invention.

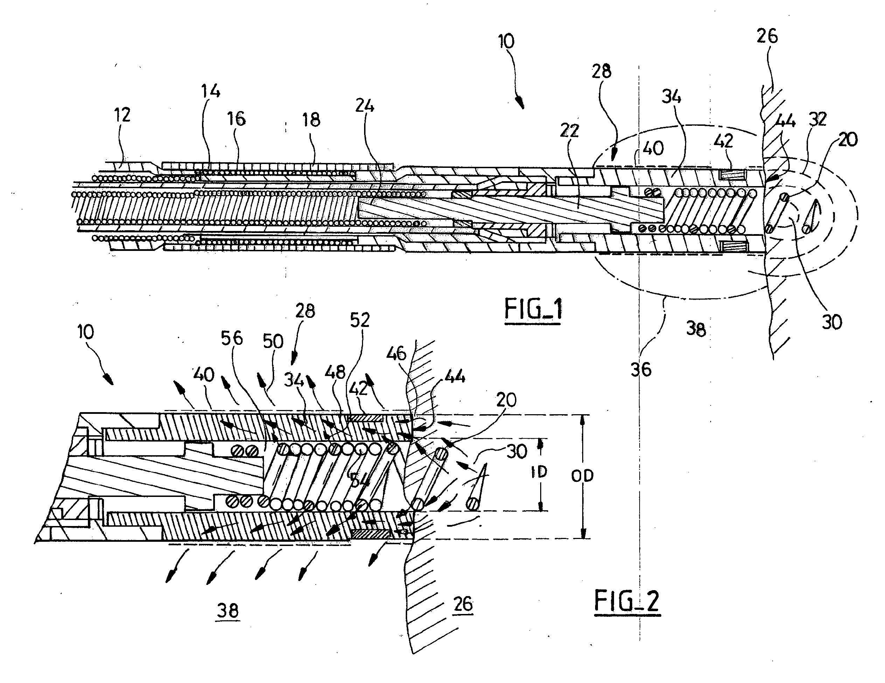

Sur la Figure 1, on a représenté une tête de sonde 10 d'une sonde du type à vis rétractable, en situation avec la vis d'ancrage en position déployée, ancrée dans les tissus de la paroi cardiaque.

Cette tête de sonde 10 est montée à l'extrémité d'une gaine 12 avec laquelle elle constitue le corps de sonde. La gaine 12 a la forme d'un tube creux flexible incorporant deux (ou plus) conducteurs électriques 14, 16, reliés à des électrodes respectives. Le conducteur 14 est relié à une électrode annulaire proximale 18 ou *ring*, et le conducteur 16 est relié à la vis d'ancrage 20, par l'intermédiaire d'un équipage mobile 22 terminé en partie arrière par une queue 24 reliée électriquement et mécaniquement au conducteur 16 de manière à assurer la continuité électrique depuis ce conducteur jusqu'à la vis 20 située à l'extrémité opposée.

La tête de sonde comporte un mécanisme (non représenté en détail) de déploiement de la vis 20 de manière que, une fois déployée, celle-ci puisse venir s'ancrer dans la paroi 26 de l'endocarde, pour assurer une liaison mécanique avec les tissus du myocarde et empêcher tout déplacement ou délogement de la tête de sonde 10 une fois celle-ci en place. La vis 20 est en outre une "vis active", jouant le rôle d'électrode de détection/stimulation par sa liaison au générateur via le conducteur 16.

La vis 20 et son mécanisme de déploiement sont logés dans un élément rigide 28 de la tête de sonde, dénommé généralement "*housing*" ou boîtier, de forme tubulaire.

Pour augmenter l'efficacité de la vis, celle-ci n'est rendue conductrice que sur la partie extrême de son extrémité distale, au voisinage de la pointe, avec une surface de contact réduite, typiquement de l'ordre de 2 mm². Pendant un examen IRM, les ondes RF captées par le corps de sonde créent une circulation de courant ayant pour effet une élévation de température à l'extrémité distale, tout particulièrement dans la région 30 au centre de la partie conductrice de l'extrémité distale de la vis 20. Compte tenu de la surface de contact réduite, la densité de courant à cet endroit est particulièrement élevée, provoquant un échauffement local important des tissus qui, s'il se prolonge, peut entraîner une destruction partielle ou totale des cellules, comme on l'a expliqué en introduction. Cet échauffement est symbolisé par les lignes 32 de la région centrale 30 de la vis. L'invention propose, pour diffuser et évacuer la chaleur ainsi générée, de réaliser le boîtier 28, au moins dans la partie la plus distale, avec une partie massive 34 en matériau thermiquement conducteur, de manière à pouvoir drainer vers la région proximale la chaleur générée dans la zone 30, afin de diffuser cette chaleur dans la zone symbolisée par le contour 36 et l'évacuer dans le flux sanguin (volume 38) entourant la partie massive 34.

On notera à cet égard que la partie massive 34 est un élément monobloc constituant le boîtier ou *housing* de la région distale de la tête de sonde, sans solution de continuité entre la face de contact 44 et la surface extérieure cylindrique baignant dans le flux sanguin 38, ni entre la surface intérieure du logement de la vis d'ancrage et cette même surface cylindrique baignant dans le flux sanguin 38. Il est en effet important que la transmission de chaleur se fasse d'une façon qui ne soit pas interrompue par un matériau formant barrière thermique, qu'il s'agisse d'air ou d'un isolant électrique.

En drainant la chaleur vers l'arrière de la zone 30 où elle prend naissance, on limite ainsi l'échauffement dans cette région et dans les tissus avoisinants du myocarde.

La Figure 2 est une vue agrandie de la partie droite de la Figure 1, montrant la manière dont s'opère le transfert de chaleur au sein de l'extrémité distale de la sonde.

Sur cette figure, on a désigné par la référence 44 la surface d'extrémité frontale de la partie massive 34, qui est une surface plane circulaire venant en contact avec la paroi 26 de l'endocarde. Les dimensions typiques de la surface d'appui sont de l'ordre de 5 à 7 French (1,66 à 2,33 mm) pour le diamètre extérieur *OD* et 1,2 à 2 mm pour le diamètre intérieur *ID*). La chaleur générée dans la région 30 au centre de la partie conductrice de l'extrémité distale de la vis 20 est transférée à la partie massive 34 via cette face de contact 44, qui joue le rôle de pont thermique entre l'endocarde 26 et la partie massive 34.

La chaleur se diffuse ainsi essentiellement en direction axiale au travers de l'interface entre la face de contact 44 et l'endocarde 26 (flèches 46), puis dans la masse de la partie massive 34 (flèches 48), qui cède ensuite cette chaleur au volume environnant 38 du flux sanguin (flèches 50).

Le transfert de chaleur depuis la région 30 où prend naissance l'échauffement vers le volume du flux sanguin 36 se fait également par l'intermédiaire de la vis 20 elle-même, via les spires de celle-ci en contact avec la paroi interne de la partie massive cylindrique 34 (flèches 52).

Ainsi, du point de vue de la conduction de la chaleur, la partie massive 34 est mise en communication avec la région 30 de l'échauffement via deux ponts thermiques distincts, constitués par (i) la face d'appui 44 et (ii) la vis d'ancrage 20.

Pour favoriser le transfert thermique via la vis d'ancrage 20, il est avantageux de prévoir un élément thermiquement conducteur spiralé 54 logé entre les spires de la vis 20, de manière à obtenir un ensemble à spires jointives. Cet élément spiralé additionnel 54 a pour fonction de remplir l'espace vide entre les spires de la vis 20 et d'augmenter ainsi la masse de matériau susceptible de drainer la chaleur vers la partie massive 34. Il peut notamment être réalisé en titane ou, idéalement, en platine (pour bénéficier de la conductivité thermique très supérieure de ce dernier matériau).

De façon également avantageuse, un gel conducteur thermique peut être introduit dans le volume compris entre la vis d'ancrage 20 et le diamètre interne de la partie massive 34 (espace vide référencé 56 sur la Figure 2). La présence d'un tel gel conducteur thermique permet d'éliminer tout film d'air (très résistant au transfert de chaleur) entre la vis d'ancrage et la paroi intérieure de la partie massive 34, ceci afin de garantir, ici encore, un transfert de chaleur optimum en direction radiale.

Ce gel peut être notamment un hydrogel se présentant avant implantation sous forme sèche, et qui se réhydratera instantanément au contact de l'eau ou du sang. Cette hydrogel peut être notamment un gel de PVP (polyvinylpyrrolidone), qui présente sous forme hydratée des caractéristiques thermiques proches de l'eau (qui est son principal constituant, sous cette forme). L'autre avantage du gel de PVP est sa biocompatibilité très bien documentée.

Le choix du matériau de la partie massive thermiquement conductrice 34 nécessite la prise en compte des niveaux de conductivité thermique typiques des différents matériaux susceptibles d'être rencontrés ou utilisés. Ce paramètre est indiqué dans le Tableau 1 ci-dessous pour divers matériaux connus.

**Tableau 1**

| Matériau | Conductivité thermique (W/mK) |
|---|---|
| Air | 0,03 |
| Silicone | 0,11 |
| Parylène | 0,14 |
| PEEK | 0,25 |
| Sang | 0,50 |
| Muscle | 0,54 |
| Eau | 0,60 |
| Carbone | 4 à 6 |
| Titane | 7,50 |
| Acier inox | 26,00 |
| Platine | 71,60 |
| Diamant | 1000 à 2600 |

Les matériaux habituellement utilisés jusqu'à présent pour réaliser le boîtier 34 de la tête de sonde, tels que silicone, parylène, PEEK, présentent tous une conductivité thermique très faible, très inférieure à 1, ce qui produit un effet de barrière thermique empêchant la diffusion de la chaleur générée pendant un examen IRM au niveau de la partie active de la pointe de vis.

Pour annihiler cet effet de barrière thermique, et le remplacer au contraire par un effet de pompe ou drain thermique, l'invention propose de choisir un matériau présentant une conductivité thermique beaucoup plus élevée, typiquement d'au moins 5.

Compte tenu des contraintes spécifiques de biocompatibilité, il est possible de choisir ainsi pour la partie massive 34, en remplacement du silicone ou du PEEK, un métal tel que le titane, qui présente l'avantage d'être radio-transparent, et donc de conserver le caractère fonctionnel des marqueurs radio-opaques permettant au chirurgien de contrôler le déploiement de la vis sous amplificateur de brillance.

Il est néanmoins indispensable d'isoler électriquement la surface externe de la partie massive 34 en titane.

Dans l'état de la technique, on sait isoler des pièces en titane par un revêtement d'un matériau tel que le parylène, mais le tableau plus haut indique que la conductivité thermique de cette matière est très faible.

Afin de maintenir l'efficacité du pont thermique, l'invention propose de remplacer le revêtement parylène conventionnel par un dépôt de surface en un matériau de conductivité thermique élevée, en particulier par un dépôt de type diamant.

Ce matériau est particulièrement intéressant dans la présente application, car il combine une très forte conductivité thermique (1000 à 2600) à une capacité d'isolement électrique satisfaisante.

Le dépôt d'un revêtement en diamant sur un cylindre métallique est une technologie en elle-même connue, mais elle avait été jusqu'à présent essentiellement utilisée pour bénéficier des propriétés de très faible coefficient de frottement du diamant, et donc faciliter le glissement lors des manoeuvres d'introduction, mais n'avait jamais été proposée pour tirer parti de sa conductivité thermique exceptionnellement élevée.

Le revêtement isolant a été schématisé en 40 sur la Figure 1, et il est présent sur toute la longueur de la partie massive en matériau thermiquement et électriquement conducteur 34, à l'exception éventuelle d'une région logeant un collier 42 de diffusion d'un stéroïde, à proximité de la zone de contact avec la paroi cardiaque 26.

Le revêtement en diamant peut être réalisé non seulement sur la partie massive et thermiquement conductrice 34 du boîtier 28 enfermant la vis et son mécanisme, mais également sur la vis elle-même, à l'exception bien entendu d'une surface non revêtue à l'extrémité de la pointe, pour maintenir la fonction de base de vis active (correspondant sensiblement aux deux derniers millimètres en direction distale).

La configuration inverse est également envisageable, où le diamant isolant est déposé sur la pointe de la vis et le reste de la vis active étant laissé non revêtu. Cette dernière configuration présente l'avantage d'éviter des densités de courants élevées à la pointe de vis pouvant provoquer localement un échauffement plus intense, d'autant plus gênant qu'il est mal drainé thermiquement par les faibles surfaces de conduction que présente une pointe. De fait, si la pointe est isolée et le corps de la vis est dénudé, l'échauffement peut être mieux réparti le long de la vis et également mieux drainé.

## Revendications

1. Une sonde intracardiaque de stimulation ou de défibrillation, comportant une gaine souple tubulaire (12) terminée à son extrémité distale par une tête de sonde (10) comprenant :
- un boîtier extérieur tubulaire (28) au moins en partie électriquement isolant;
- un moyen d'ancrage de la tête de sonde, relié au boîtier tubulaire et comprenant une vis d'ancrage hélicoïdale saillante (20) prolongeant axialement le boîtier tubulaire, cette vis étant une vis active, électriquement conductrice sur au moins une partie d'extrémité, formant ladite électrode de stimulation;
- une électrode de stimulation distale, disposée à l'extrémité de la tête de sonde ; et
- un élément formant drain thermique, électriquement isolé et porté par le boîtier tubulaire (28) dans la région extérieure de celui-ci, comprenant une partie massive (34) en un matériau thermiquement conducteur, qui s'étend en direction axiale jusqu'à l'extrémité distale de la tête de sonde, et est terminée par une face frontale de contact (44) apte à venir en contact avec une paroi musculaire (26) du patient pour former un pont thermique entre cette paroi et la partie massive,
**caractérisée en ce qu'**elle comprend en outre un élément spiralé (54), réalisé en un matériau thermiquement conducteur, inséré entre les spires de la vis d'ancrage hélicoïdale (20) de manière à remplir l'espace libre existant entre ces spires.

2. La sonde de la revendication 1, dans laquelle le moyen d'ancrage comprend des barbes d'ancrage faisant saillie radialement vers l'extérieur du boîtier tubulaire, et l'électrode de stimulation distale est une électrode apte à venir en appui contre une paroi musculaire.

3. La sonde de la revendication 1, dans laquelle la face frontale de contact (44) est une face radiale plane formant l'extrémité distale du boîtier tubulaire (28).

4. La sonde de la revendication 1, dans laquelle le boîtier extérieur tubulaire comprend un logement interne pour une extrémité proximale du moyen d'ancrage, et dans lequel ladite partie massive de l'élément formant drain thermique s'étend en direction radiale sans solution de continuité depuis le logement jusqu'à la paroi extérieure libre du boîtier.

5. La sonde de la revendication 4, comprenant un matériau thermiquement conducteur de remplissage de la cavité, de type gel.

6. La sonde de la revendication 5, dans laquelle le matériau de remplissage de la cavité est un hydrogel réhydratable.

7. La sonde de la revendication 1, dans laquelle le matériau thermiquement conducteur de la partie massive présente une conductivité thermique d'au moins 5 W/mK.

8. La sonde de la revendication 1, dans laquelle le matériau thermiquement conducteur de la partie massive est un matériau radio-transparent.

9. La sonde de la revendication 1, dans laquelle le matériau thermiquement conducteur de la partie massive est un matériau métallique, et la partie massive porte en surface un revêtement (40) électriquement isolant

10. La sonde de la revendication 9, dans laquelle le matériau métallique est le titane ou un alliage de titane.

11. La sonde de la revendication 9, dans laquelle le revêtement électriquement isolant est en un matériau thermiquement conducteur.

12. La sonde de la revendication 11, dans laquelle le revêtement électriquement isolant en matériau thermiquement conducteur de la partie massive est un dépôt de diamant.

13. La sonde de la revendication 1, dans laquelle la vis est pourvue, sur une partie de sa longueur, d'un revêtement électriquement isolant en un matériau thermiquement conducteur.

14. La sonde de la revendication 13, dans laquelle le revêtement électriquement isolant en matériau thermiquement conducteur de la partie de vis est un dépôt de diamant.

## Patentansprüche

1. Intrakardiale Stimulations- oder Defibrillationssonde, die eine rohrförmige biegsame Hülle (12) aufweist, welche an ihrem distalen Ende in einem Sondenkopf (10) endet, der enthält:
- ein zumindest teilweise elektrisch isolierendes rohrförmiges Außengehäuse (28);
- eine Verankerungseinrichtung des Sondenkopfs, die mit dem rohrförmigen Gehäuse verbunden ist und eine vorstehende Spiralankerschraube (20) enthält, die das rohrförmige Gehäuse axial verlängert, wobei diese Schraube eine in mindestens einem Endbereich elektrisch leitende aktive Schraube ist, die die Stimulationselektrode bildet,
- eine distale Stimulationselektrode, die am Ende des Sondenkopfs angeordnet ist; und
- ein eine Wärmesenke bildendes Element, das elektrisch isoliert ist und vom rohrförmigen Gehäuse (28) in dessen Außenbereich getragen wird, das einen massiven Teil (34) aus einem wärmeleitenden Material enthält, der sich in axialer Richtung bis zum distalen Ende des Sondenkopfs erstreckt und in einer Kontaktstirnseite (44) endet, die mit einer Muskelwand (26) des Patienten in Kontakt kommen kann, um eine Wärmebrücke zwischen dieser Wand und dem massiven Teil zu bilden,
**dadurch gekennzeichnet, dass** sie außerdem ein aus einem wärmeleitenden Material hergestelltes Spiralelement (54) enthält, das so zwischen die Windungen der Verankerungsschraube (20) eingefügt ist, dass es den zwischen diesen Windungen existierenden Freiraum füllt.

2. Sonde nach Anspruch 1, wobei die Verankerungseinrichtung Verankerungswiderhaken enthält, die radial nach außerhalb des rohrförmigen Gehäuses vorstehen, und die distale Stimulationselektrode eine Elektrode ist, die gegen eine Muskelwand in Auflage kommen kann.

3. Sonde nach Anspruch 1, wobei die Kontaktstirnseite (44) eine ebene radiale Seite ist, die das distale Ende des rohrförmigen Gehäuses (28) bildet.

4. Sonde nach Anspruch 1, wobei das rohrförmige Außengehäuse eine innere Aufnahme für ein proximales Ende der Verankerungseinrichtung enthält, und wobei der massive Teil des eine Wärmesenke bildenden Elements sich in radialer Richtung ohne Unterbrechung von der Aufnahme bis zur freien Außenwand des Gehäuses erstreckt.

5. Sonde nach Anspruch 4, die ein wärmeleitendes Füllmaterial des Hohlraums von der Art Gel enthält.

6. Sonde nach Anspruch 5, wobei das Füllmaterial des Hohlraums ein rehydratisierbares Hydrogel ist.

7. Sonde nach Anspruch 1, wobei das wärmeleitende Material des massiven Teils eine Wärmeleitfähigkeit von mindestens 5W/mK aufweist.

8. Sonde nach Anspruch 1, wobei das wärmeleitende Material des massiven Teils ein röntgenstrahldurchlässiges Material ist.

9. Sonde nach Anspruch 1, wobei das wärmeleitende Material des massiven Teils ein metallisches Material ist, und der massive Teil an der Oberfläche eine elektrisch isolierende Beschichtung (40) trägt.

10. Sonde nach Anspruch 9, wobei das metallische Material Titan oder eine Titanlegierung ist.

11. Sonde nach Anspruch 9, wobei die elektrisch isolierende Beschichtung aus einem wärmeleitenden Material ist.

12. Sonde nach Anspruch 11, wobei die elektrisch isolierende Beschichtung aus wärmeleitendem Material des massiven Teils eine Diamantabscheidung ist.

13. Sonde nach Anspruch 1, wobei die Schraube über einen Teil ihrer Länge mit einer elektrisch isolierenden Beschichtung aus einem wärmeleitenden Material versehen ist.

14. Sonde nach Anspruch 13, wobei die elektrisch isolierende Beschichtung aus wärmeleitendem Material des Schraubenteils eine Diamantabscheidung ist.

## Claims

1. An endocardial stimulation or defibrillation lead, including a flexible tubular sheath (12) terminated at its distal end by a lead head (10) comprising:
- a tubular external housing (28) at least partially electrically insulating;
- a means for anchoring the lead head, connected to the tubular housing and comprising a protruding helical anchoring screw (20) continuing axially the tubular housing, this screw being an active screw, electrically conductive over at least an end portion, forming said stimulation electrode;
- a distal stimulation electrode, arranged at the end of the lead head; and
- a thermal-drain element, electrically insulated and carried by the tubular housing (28) in the external region of the latter, comprising a massive portion (34) made of a thermally conductive material, which extends axially to the distal end of the lead head, and is terminated by a contact front face (44) adapted to come into contact with a muscular wall (26) of the patient to form a thermal bridge between this wall and the massive portion,
**characterized in that** it further comprises a spiral element (54), made of a thermally conductive material, inserted between the threads of the helical anchoring screw (20) so as to fill the free space existing between these threads.

2. The lead of claim 1, wherein the anchoring means comprises anchoring barbs protruding radially outwardly from the tubular housing, and the distal stimulation electrode is an electrode adapted to come in rest against a muscular wall.

3. The lead of claim 1, wherein the contact front face (44) is a planar radial face forming the distal end of the tubular housing (28).

4. The lead of claim 1, wherein the tubular external housing comprises an internal accommodation for a proximal end of the anchoring means, and wherein said massive portion of the thermal-drain element extends radially without discontinuity from the accommodation to the free external wall of the housing.

5. The lead of claim 4, comprising a cavity-filling thermally-conductive material of the gel type.

6. The lead of claim 5, wherein the cavity-filling material is a rehydratable hydrogel.

7. The lead of claim 1, wherein the thermallyconductive material of the massive portion has a thermal conductivity of at least 5 W/mK.

8. The lead of claim 1, wherein the thermallyconductive material of the massive portion is a radiotransparent material.

9. The lead of claim 1, wherein the thermallyconductive material of the massive portion is a metal material, and the massive portion has on its surface an electrically insulating coating (40).

10. The lead of claim 9, wherein the metal material is the titanium or an titanium alloy.

11. The lead of claim 9, wherein the electrically insulating coating is made of a thermally conductive material.

12. The lead of claim 11, wherein the electrically insulating coating, made of a thermally conductive material, of the massive portion is a diamond deposit.

13. The lead of claim 1, wherein the screw is provided, over a portion of its length, with an electrically insulating coating made of a thermally conductive material.

14. The lead of claim 13, wherein the electrically insulating coating, made of a thermally conductive material, of the screw portion is a diamond deposit.
